(19) **Europäisches Patentamt**
**European Patent Office**
**Office européen des brevets**

(11) **EP 3 270 355 B1**

(12) **EUROPEAN PATENT SPECIFICATION**

(45) Date of publication and mention
of the grant of the patent:
**31.07.2019 Bulletin 2019/31**

(21) Application number: **17153565.1**

(22) Date of filing: **27.01.2017**

(51) Int Cl.:
*G06T 7/60* (2017.01)　　　*G06T 11/00* (2006.01)
*G06T 7/00* (2017.01)　　　*A61B 6/00* (2006.01)
*A61B 5/00* (2006.01)　　　*A61B 5/055* (2006.01)
*A61B 5/026* (2006.01)　　　*A61B 5/02* (2006.01)

(54) **DETERMINING A COMPLEXITY VALUE OF A STENOSIS OR A SECTION OF A VESSEL**

BESTIMMUNG EINES KOMPLEXITÄTSWERTES EINER STENOSE ODER EINER SEKTION EINES GEFÄSSES

DÉTERMINATION D'UNE VALEUR DE COMPLEXITÉ D'UNE STÉNOSE OU D'UNE SECTION D'UN VAISSEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.01.2018 Bulletin 2018/03**

(73) Proprietor: **Siemens Healthcare GmbH**
**91052 Erlangen (DE)**

(72) Inventors:
• **Berger, Martin**
**91052 Erlangen (DE)**

• **Redel, Thomas**
**91099 Poxdorf (DE)**

(56) References cited:
**US-A1- 2014 073 976　　US-A1- 2015 066 818**

• **WEI JUN ET AL: "Computerized detection of noncalcified plaques in coronary CT angiography: Evaluation of topological soft gradient prescreening method and luminal analysis", MEDICAL PHYSICS, AIP, MELVILLE, NY, US, vol. 41, no. 8, 1 August 2014 (2014-08-01) , XP012187736, ISSN: 0094-2405, DOI: 10.1118/1.4885958 [retrieved on 1901-01-01]**

**Description**

The present invention relates to a method for determining whether a further image of a stenosis or a section of a vessel should be provided or not based on evaluating a geometrical complexity of a stenosis or a section of a vessel. Furthermore, the present invention relates to a corresponding device for determining whether a further image of a stenosis or a section of a vessel should be provided or not based on evaluating such geometrical complexity. Additionally, a corresponding angiography system is presented (for simplicity reasons the word angiography is also used in the meaning arteriography in the present document).

Usually, stents are used for treating coronary arteries being constricted by plaque in order to re-establish the blood flow and thus the supply of the cardiac muscle. A 3D-reconstruction of a blood vessel model can be used for estimating the degree of stenosis as well as for planning a treatment strategy. For this purpose, a 3D-blood-vessel model is developed based on at least two angiographic scenes. The precision of the reconstructed blood vessel model is decisive, since it directly affects the evaluation of the stenosis or the section of the vessel. In the present document for simplicity reasons the word "stenosis" is used for both: the literal meaning of a stenosis and the meaning of a section of a vessel, as far as the second meaning is not explicitly mentioned.

Due to the limited number of angiographic pictures, the geometric complexity of the 3D-models is usually restricted to elliptical cross-sections, which are located on a reconstructed 3D-center-line. The re-construction of the models are based on 2D-segmentations of a vessel contour and its center line. The effects of stenoses on the 2D-contour of the vessel strongly depends on the complexity of the stenoses, but also on the perspective of the single views.

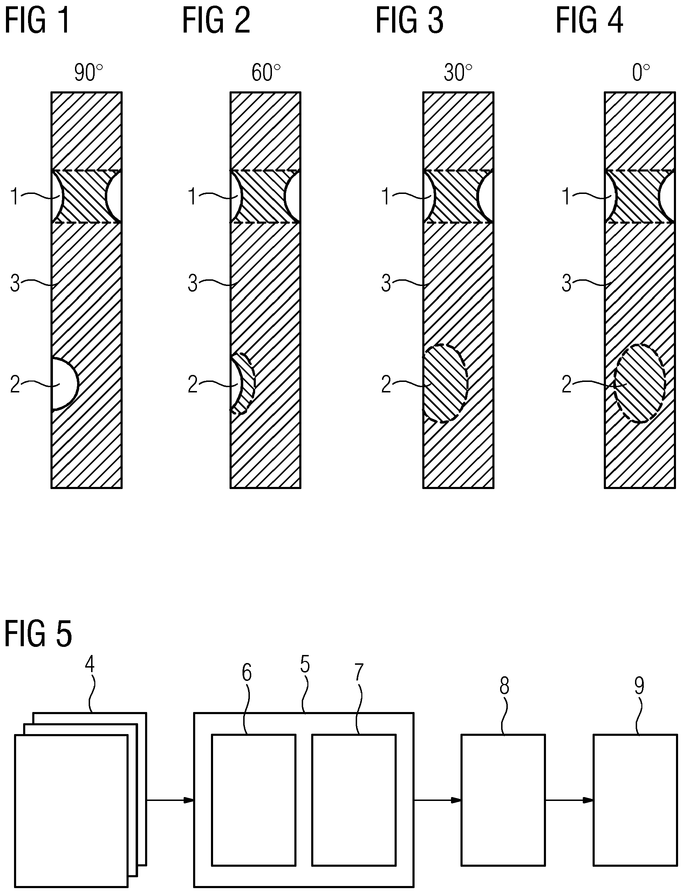

FIG 1 to 4 illustrate the problem. A metal phantom is used to model an axisymmetric stenosis 1 and an eccentric stenosis 2. Projection images show significant differences of the contours of both stenoses 1 and 2.

Figures 1 to 4 show, for example, different LAO (left anterior oblique) views. FIG 1 is a view at LAO = 90°, FIG 2 a view at LAO = 60°, FIG 3 a view at LAO = 30° and FIG 4 a view at LAO = 0°. The geometrical contour of the axisymmetric stenosis 1 is equally formed in each of the views of FIG 1 to 4. However, the contour of the eccentric stenosis 2 can only be seen in the views of FIG 1 and 2. I.e. only the views at LAO = 90° and LAO = 60° show a crescent shape at the edge of the vessel 3 (represented by the metal phantom). Since the eccentric stenosis does not cover the complete circumference of the vessel, it is almost completely hidden in unfavorable views like LAO = 0° and LAO = 30° (compare FIG 3 and 4). These views do not allow a contour based reconstruction of the eccentric stenosis 2.

A solution of this problem would be to take additional pictures at further angles which provide important information for improving the 3D-model. Such additional pictures, however, have the disadvantage that they increase the time and consequently the costs for acquisition and processing of the pictures. Moreover, the dose of radiation increases for the patient and the cardiologist. Therefore, additional pictures should only be taken if the complexity of the stenosis requires them.

If the stenosis is diagnosed directly from 2D-angiography data, the number of pictures as well as their views are manually assessed by the physician. Furthermore, there is no automated solution for the evaluation of the complexity and the reconstructability of the stenosis based on the 2D-angiography data.

The article of Itu, Lucian, et al.: "A Machine Learning Approach for Computation of Fractional Flow Reserve from Coronary Computed Tomography", Journal of Applied Physiology, April 14, 2016, presents a machine learning based model for predicting FFR (fractural flow reserve) as an alternative physician-based approach. The model is trained on a large data base of synthetically generated coronary anatomies, where the target values are computed using the physician-based model. The trained model predicts FFR at each point along the center line of the coronary tree and its performance was assessed by comparing the predictions against physician-based computations.

The article of Jun Wei et al.:"Computerized detection of noncalcified plaques in coronary CT angiography: Evaluation of topological soft gradient prescreening method an luminal analysis", Medical Physics, AIP, Melville, NY, US, vol. 41, no. 8, 1 August 2014, ISSN: 0094-2405 discloses the detection on noncalcified plaques in coronary arteries. Luminal analysis is performed using 3D geometric features and gray-level features.

The object of the present invention is to provide a method of evaluating a geometrical complexity of a stenosis which could be used as basis for deciding whether further images of the stenosis should be gathered for performing a 3D-reconstruction of the stenosis, for example. Furthermore, a respective device shall be provided.

According to the present invention this object is solved by a method according to claim 1 and a device according to claim 11. Further favorable developments are defined in the subclaims.

The geometrical complexity relates to the geometrical form of the stenosis or the section of the vessel. The more complex a stenosis or vessel is, the more images of the stenosis or vessel may be necessary to reconstruct the stenosis or vessel with high quality in three dimensions. For the inventive method, at least one image of the stenosis or the section of the vessel is necessary as starting point. At least one geometrical feature value is obtained from this image. This geometrical feature value may be a value representing the diameter, the length or any other geometrical parameter of the stenosis or the section of the vessel. For higher performance a plurality of geometrical feature values may be gathered. Individual complexity values may be gathered along the centerline of vessel.

Additionally, at least one intensity feature value is obtained or determined from the image. The intensity feature value relates to a grey level intensity of a region of the image which shows the stenosis. The intensity reflects e.g. the attenuation of the (X-) rays for obtaining the picture or image.

The process of extracting geometrical or intensity-based features may also include one or multiple preprocessing steps on the projection image. For example, a vesselness filter or other gradient-based methods may be applied to enhance the vessel structures and suppress the background anatomy.

In a preferred embodiment multiple features may be combined to determine a complexity value $\mu$. An alternative geometrical contribution may be provided by a comparison of the two outlines of the stenosis. In a preferred manner the amount of correlated curvature k1 and k2 of both outlines from both side of the vessel segment containing the stenosis may be compared normalized by calculating a ratio or difference or more complex coefficients, for example:

$$\mu K = k1 - k2; \quad \text{or } K = k1 \times k2$$

The complexity value related to the geometrical complexity of the stenosis is determined in an automatical manner. This means that no physician needs to assess the complexity of the form of the stenosis, rather an automatic algorithm is used to determine the complexity of the geometrical form of the stenosis. This algorithm uses the at least one geometrical feature value and the at least one intensity feature value of the stenosis as input values. In a simple configuration there are only two different values to determine the complexity value, namely one geometrical feature value and one intensity feature value. In high-end configurations there may be used a plurality of geometrical feature values and a plurality of intensity feature values as a vector for automatically determining the complexity value.

The advantage of the inventive method is that two different types of feature values are employed for evaluating the geometrical complexity of the stenosis. Additionally, plural subtypes of these feature types can be used for determining or estimating the complexity. Thus, a big parameter room can be utilized in order to obtain a simple but significant complexity value for the stenosis.

In one embodiment the at least one image may be provided by angiography. However, also other imaging techniques may be used for gathering the at least one image. Specifically, X-ray techniques or magnet resonance tomography may be used to obtain the images/pictures.

The at least one geometrical feature value may relate to one of a 2D-contour, a center line of a blood vessel or a curvature of a blood vessel. The 2D-contour may be obtained by an edge filter applied to the image. The 2D-contour may reflect the form of the stenosis. Alternatively, the geometrical feature value may relate to the center line of a blood vessel. Specifically, the distance of the stenosis from the center line may be of interest. This distance may vary along the center line. As a further geometrical feature, the curvature of the blood vessel may be of importance. In this case the feature value may relate to the radius of curvature of the blood vessel. Even other simple geometrical feature values like diameter, length and so on can be used for determining the complexity of the stenosis or the vessel along its centerline, for example. Additionally, further processing functions like derivation, Fourier transformation, wavelet analysis etc. may be employed for determining the complexity value. Beside of that position information of the centerline of the vessel or the lumen may contribute to the complexity value.

Preferably, the at least one intensity feature value for determining the complexity of the stenosis results from an attenuation or excitation of radiation and relates to a sum, a distribution or an energy of gray values within one or more regions of the blood vessel. For instance, the attenuation of radiation may be of interest when performing an X-ray screening. On the other hand, the excitation of radiation is used in MRT-screenings. Both the local attenuation and local excitation of radiation lead to locally different gray values of an image, and these gray values can be further processed to obtain one or more intensity feature values.

The complexity value of the stenosis may be determined by regression or classification of the at least one geometrical feature value and the at least one intensity feature value. This means that two or more values are provided as input values for a regression or classification algorithm in order to obtain the complexity value. Any type of regression algorithm and/or classification algorithm may be implemented. The complexity of the stenosis thus can be advantageously described with one single value. Of course, the complexity could also be defined by a plurality of values. The one or more complexity values may be provided for one position or plural positions along a centerline, for instance.

Preferably, two, three or more values of one or more geometrical features and one or more intensity features are gathered and the complexity value is determined by rating the feature values. For example, there is formed a vector of two, three or more values. Each value relates to a geometrical feature or an intensity feature. The vector includes at least one geometrical feature value and at least one intensity feature value. For determining the complexity value of the stenosis each value of the vector is rated. Such rating of the plural values represents a certain kind of regression.

The geometrical area might be estimated herein by the local diameter measured while the intensity might be estimated by using Beers law and with the healthy segment used as reference for calibration. The area information can be used to derive symmetric r or hydraulic radii R for comparison. In concrete there are different ways to define complexity μ. In a first simple approach it is the difference or ratio of the diameters at the maximum stenosis measured on geometrical Ageo(stenosis) and intensity Aint (stenosis) based measurement. Complexity might for example be determined as:

μ= Ageo/ Aint ; or p=Ageo - Aint; or μ = (Ageo - Aint)/ Ageo or
μ= Rgeo/ Rint ; or μ=Rgeo - Rint; or μ = (Rgeo - Rint)/ Rgeo or
μ= rgeo/ rint ; or μ=rgeo - rint; or μ = (rgeo - rint)/ rgeo

Complexity μ can be determined at multiple locations A(x) along a stenosis with length l. Based on that multiple numbers an average, integrated, median, added, multiplied or a combination, single value can be obtained in the geometrical space as well as in the intensity based space. Alternatively instead of multiple locations complete areas can be used. Complexity μ using multiple locations might for example be determined as:

$$\mu tot \ = \ \int_{0}^{l} \mu(x) dx \ ;$$

or

$$\mu tot \ = \ \mu(1) \ x \ \mu(2) \ x \ \mu(3) \ ...;$$

or

$$\mu tot \ = \ median(\mu(x));$$

or

$$\mu tot \ = \ \Sigma \frac{Ageo(x) \cdot dx}{Aint(x) \cdot dx}$$

Further geometrical features may be the centerlines, or the curvature c of the centerline, calculated as measured geometrical feature or derived from the maximum of the intensity based distribution. In the latter case the location of the centerline in a cross section is defined by the maximum intensity along the cross section. Also here different mathematical operations are possible to compare both values to a complexity value μC, for example:

$$\mu C \ = \ cgeo;$$

or

$$\mu C \ = \ cint \ \ (geometrical \ or \ intensity \ based \ only);$$

or preferred in combined manner

$$\mu C \ = \ cgeo \ - \ cint; \ \mu C \ = \ cgeo \ x \ cint$$

In a preferred step the different features may be combined to generate an overall complexity measure:

$$\mu tot \ = \ \mu tot(A) \ \otimes \ \mu K \ \otimes \ \mu C \ \otimes \ additional \ features$$

with μtot(A)geometrical and intensity based, μK and μC intensity and/ or geometrical based.

In a first image the analysis of the complexity $\mu$ is limited to this first image. After the second image the analysis can be performed in each of the available images. In addition if there are an initial 3D reconstruction of the vessel segment with the stenosis both analyses are registered and can be analyzed in a combined way pointwise for each location of the stenotic segment and/ or globally, for example:

$$\mu 3d = \mu image1 \otimes \mu image2$$

Specifically, a ratio of the at least one geometrical feature value and the at least one intensity feature value may be used for determining the complexity value. For example, a cross-sectional area of the vessel at the stenosis as geometrical feature value may be divided by a medium-gray level at the stenosis as intensity feature value in order to obtain the complexity value as quotient. Thus, the complexity value can be obtained very easily.

In a preferred embodiment the automatically determining of the complexity value is performed on the basis of machine learning. For example, weights for rating the multiple feature values may be learned automatically. On the other hand a classification algorithm may be learned automatically. Thus, high quality of the complexity value can be expected.

A large variety of possible features exist, e.g. geometrical or intensity features. Methods of machine learning provide a suitable framework to learn and extract stenotic complexity or reconstructability. In the following we provide a detailed example of how such a machine learning-based approach may be used.

As input to this approach we assume to have N known 3D volumetric data of contrast filled vessel segments or vessel trees. The data may be obtained by CTA, coronary MRI, or other coronary imaging modalities, yet, also simulated vessel anatomies are possible. Additionally, we obtain or have access to N 3D surface models or volumetric segmentations of the vessels, corresponding to the known 3D volumes.

In general, the vessel complexity or reconstructability is defined as a single or multiple quantitative measures which can be directly extracted from the known 3D vessel data. These quantitative values are then used as ground truth for training of the machine learning algorithm. Multiple such values may also be assigned as ground truth to particular locations within the known vessel segments or trees, e.g., along locations along the vessels' centerlines.

In the following a precise definition for extraction of a reconstructability measure is provided.

First, a large number of K simulated projection images are generated from of the known 3D volumetric vessel data. Afterwards, all possible combinations of M selected projection images are used as input for a given symbolic reconstruction algorithm (e.g., IZ3D), where M = 1,2,...,K. Afterwards, every obtained symbolic 3D reconstruction is compared with respect to its similarity to the known 3D surface model or segmentation. This similarity measure is identical to the vessel complexity or reconstructability and can be given by:

1) The computation of a DICE score, which measures the overlap of the known 3D segmentation of the vessel and the binarized symbolic reconstruction. It evaluates to 1 if the segmentations are identical and to 0 if they do not overlap at all.
2) The Hausdorff distance, measuring the distance of between the known, 3D surface model and the reconstructed 3D surface model.
3) Any other similarity metric defined between the reconstructed symbolic 3D data and the known, 3D vessel data.
4) All measures may also be obtained locally, leading to a multitude of complexity or reconstructability values that may be assigned to specific vessel locations, e.g., along the centerline.

In addition to the symbolic 3D reconstruction, geometric and intensity features are extracted from the M selected 2D projection images. Geometric features may be represented but are not limited to the radius, curvature, length, cross section area, of the segmented vessel. But also variations and combinations of such measures along the centerline, e.g., determined by the Fourier transformation, wavelet analysis, derivatives of different orders, integration, or multiplication, may be employed. Intensity features may be represented but are not limited to the sum, distribution, moments of gray values within a region of the vessel, e.g. along a line orthogonal to the 2D centerline but within the vessel outline. Note, that M itself can represent a feature value.

Based on the ground truth reconstructability or complexity index, a machine learning regression or classifier can be trained, i.e., weights and combinations of features are determined.

In the actual application phase, the known 3D volumetric data is no longer necessary. The same features are extracted from an acquired or loaded angiographic image as obtained for the training phase. Afterwards, the machine learning algorithm applies its learned weights and combinations to the extracted features such that an estimate of the reconstructability or complexity index is computed. For example, this measure can be used to decide if the reconstruction quality will be sufficient or if it needs improvement, e.g. by an additional acquired view.

In one embodiment the stenosis is an eccentric stenosis. Thus, the stenosis cannot be seen directly from all sides of the blood vessel. Typically for an eccentric stenosis, the visible area of the stenosis changes with the angle of view whereas the intensity value of the stenosis region remains constant for different angles of view. Thus, with both values the complexity or the type of the stenosis may be determined.

Preferably, the complexity value may be used to determine, whether a 3D-reconstruction of the stenosis is possible or not. The complexity value characterizes the form or type of the stenosis. In a simple application, the complexity value is compared to a threshold. If the threshold is exceeded or fallen short a 3D-reconstruction may be not possible. A corresponding notice may be generated automatically.

Furthermore, the complexity value is used to determine whether a further image of the stenosis should be provided or not. This means that an automatical recommendation concerning the necessity of an additional image can be generated. Thus, the dose of radiation can be kept as small as possible for the patient.

The above-mentioned object is also solved by a device for evaluating a geometrical complexity of a stenosis including storage means for providing at least one image of the stenosis, analyzing means for gathering at least one geometrical feature value of the stenosis and/or a region of the vessel at the stenosis from the at least one image and for gathering at least one intensity feature value based on a grey level intensity of the stenosis from the at least one image, calculating means for automatically determining a complexity value related to the geometrical complexity of the stenosis in dependency on the at least one intensity feature value of the stenosis.

The modifications and advantages of the above-described method also apply for the inventive device. Here, the method features represent functional features of the device.

In a preferred application the inventive device is used in a angiography system.

The present invention will now be described in more detail in connection with the attached figures showing in

FIG 1     a simulation of an axisymmetrical and an eccentric stenosis at a viewing angle of 90°;

FIG 2     the two stenoses at a viewing angle of 60°;

FIG 3     the two stenoses at a viewing angle of 30°;

FIG 4     the two stenoses at a viewing angle of 0°;

FIG 5     a diagram showing a method for evaluating the complexity of a stenosis and

FIG 6     a measurement of a cross-sectional area of a stenosis based on geometrical data and intensity data.

The embodiments described hereinafter, represent preferred examples of the present invention. It has to be noted that the single features are not limited to the combinations mentioned in the following but can be realized as single features or in other combinations.

The basic idea of the inventive method is a combination of different features of the stenosis. The features originate from 2D-projection images of the blood vessel with the stenosis. The features relate to geometrical data on the one hand and data based on intensity values of the image on the other hand. The basic assumption is that pure geometrical features, like e.g. the contour of the blood vessel, strongly depend on the viewing direction and the form of the stenosis. However, the total absorption of the X-rays caused by the blood vessel predominantly does not depend on the viewing directions. This was already explained in connection with FIG 1 to 4. Though the eccentric stenosis 2 is no longer identifiable in its contour at LAO = 0° and LAO = 30°, it is recognizable in the gray values.

FIG 5 illustrates an embodiment of a method of evaluating the complexity of a stenosis. One or more angiographic images 4 are provided. A feature extraction 5 is performed on the angiographic images 4. Analyzing means may be used for performing such feature extraction. The feature extraction 5 contains two different algorithms. A first algorithm 6 for extracting geometrical features or respective values. Optionally, the first algorithm 6 may perform a segmentation of the objects in the images 4.

A second algorithm 7 of the feature extraction 5 may extract intensity features or respective values. For example, densitometry is performable with this algorithm 7.

The features or feature values obtained from the algorithms 6 and 7 or the feature extraction 5, i.e. the analyzing means, may be input to calculating means 8. The calculating means 8 may perform a regression or classification algorithm. As a result from the calculating means 8 a complexity value 9 of the stenosis may be output. This complexity value 9 may be further processed in order to provide a value related to the ability of reconstructing the stenosis, for example, a value related to the decision: "stenosis sufficiently described for reconstruction?". The value represents the corresponding decision "yes"/"no".

Geometrical features extracted by algorithm 6 may relate to the 2D-contour, the center line or the curvature of the segmented blood vessel. For instance, an edge detector is used for extracting the edges of the blood vessel or the stenosis in order to obtain the respective contour or other features of the vessel as already mentioned.

Intensity features or intensity feature values, respectively, are calculated on the basis of e.g. the attenuation of X-rays by the blood vessel filled with the contrast agent. These intensity feature values may be determined indirectly from the gray values of an angiography image. Preferable the intensity (based) features may include, for example, the sum, the distribution or the energy of the gray values within one or more regions in the blood vessel.

A value, e.g. a numerical value, may be determined from the extracted features by any kind of combination among each other. Such combination may be a certain kind of regression or classification. The determined value represents a complexity value of the stenosis or a value indicating the possibility of reconstructing the stenosis and/or the blood vessel on the basis of the evaluated images/pictures. In a simple case the regression may be a manually selected combination of features like the quotient of a local gray value sum and a vessel radius.

Another version of the calculation of the complexity value includes a direct classification based on the extracted features. The result, for example, would be the decision whether further pictures have to be taken or not. Further examples of classification are: a) Stenosis is complex: yes/no or b) diagnostic value is reliable: yes/no. In a simple case, this may be realized by a threshold decision subsequent to the regression or classification. If the resultant value lies above the threshold, taking a further picture will be suggested. Otherwise, the stenosis is considered to be sufficiently described by the present pictures.

If picture data are available, for which the complexity value has already been determined in advance, e.g. because the 3D-blood-vessel-model is already known, these data can be used as training data. Thus, regressions based on machine learning are possible which learn the weights of the individual features automatically by means of the training data. The training data might also be generated by synthetically generated blood vessel models (compare the above mentioned article of Itu, Lucian et al.).

Furthermore, the regression can be replaced, as already mentioned by a classifier, which will be directly trained with the aid of the training data. Thus, an automated system is formed, which can decide by means of the segmented 2D-angiography data whether further images have to be taken or not.

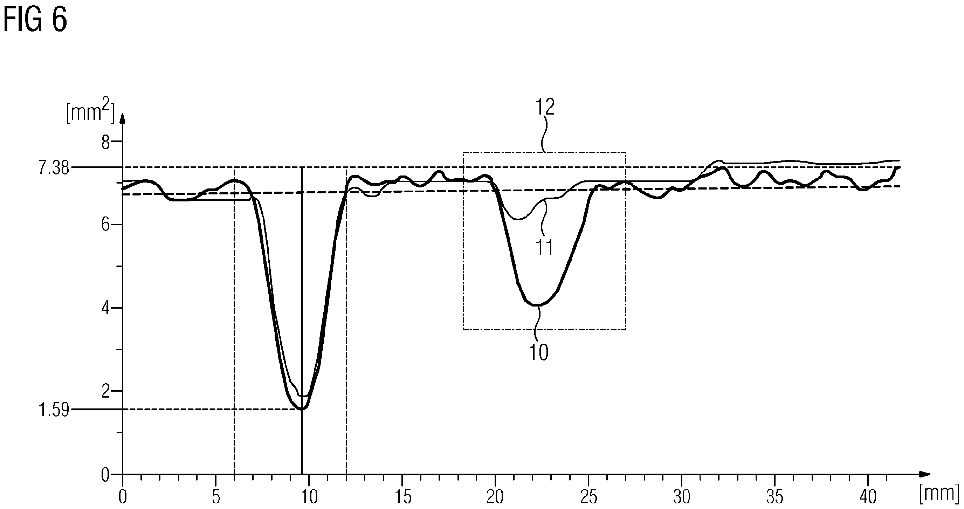

A practical example will now be described in connection with FIG 6. In the practical example a phantom emulates a blood vessel with an axisymmetrical stenosis 1 and an eccentric stenosis 2 like that of FIG 1 to 4. A 2D quantative coronary analysis (QCA) is performed. The cross-sectional area of the blood vessel is measured as geometrical feature along the center line. As intensity feature a value representing the area is extracted on the basis of the gray values within the vessel segmentation. A segmentation of the 2D-QCA may provide the contour of the stenosis and/or blood vessel. If we consider the situation of FIG 4 (LAO = 0°), where the eccentric stenosis 2 is hidden by the blood vessel 3, the symmetrical stenosis 1 can be clearly identified by its contour. In contrast, the eccentric stenosis 2 just minimally contributes to a modification of the contour of the blood vessel.

The estimated or calculated cross-sectional areas of the 2D- QCA are shown in FIG 6. Curve 10 shows the cross sectional area determined by intensity feature values and curve 11 shows the cross-sectional area of the blood vessel determined by geometrical feature values. The cross-sectional area of the symmetrical stenosis is the same when determined on the basis of the geometrical feature values or the intensity feature values. This does not apply for the eccentric stenosis which is marked by rectangle 12 in FIG 6. The estimated cross-sectional area calculated with the geometrical data has only a small drop in case of the eccentric stenosis 2, whereas the measurement of the area based on the intensity data clearly shows the eccentric stenosis 2.

A simple division of the areas obtained from geometrical data or intensity based data can be used as measure for the reconstructability or complexity of the blood vessel, including the stenosis. The result will lead to a greater drop only for the eccentric stenosis 2 which means a worse reconstructability. In this case further images might be necessary.

The complexity value may be used for a plurality of applications. Preferably, the complexity value of the stenosis is used for judging whether further angiographical images have to be taken. Furthermore, the complexity value of the stenosis can also be used as measure for the reliability of quantities obtained by angiography. Generally, plural such quantities can be determined from angiographical image data for diagnostic purposes.

If it is possible to judge by means of the complexity value whether the blood vessel is generally symmetrical, further images can be saved. In particular, when the complexity value indicates a circular vessel and a foreshortening-free acquisition of the vessel can be assumed, only a single view may be sufficient for 3D reconstruction. Moreover, the complexity value can be used as feature for manual or automatical classification, for example in a diagnostic system which decides whether a stenosis is significant or not.

A predominant aspect of the present invention is the combination of one or more geometrical feature values and one or more intensity feature values for evaluating the complexity and/or reconstructability of stenoses from angiographic scenes. A further advantage may be an automated system for the decision whether further images are necessary or not.

The described method allows for rapidly judging the reconstructability and/or complexity of a stenosis on the basis of already present angiography data. Thus, the 3D-reconstruction of complex stenoses may be performed with a minimum number of angiographic images. This saves time and resources during medical interventions and reduces the radiation dose for the patient and the physician.

Furthermore, the application of the method may lead to a minimum level of reconstruction precision. It can be guaranteed that more complex types of stenoses can be identified and reconstructed exactly (e.g. by images of additional views).

The described automation of the methods by machine learning leads to a further automation of the work flow and allows for the combination of a high number of geometrical and intensity-based features or feature values, respectively.

**Claims**

1. Method for determining whether a further image (4) of a stenosis (1,2) or a section of a vessel (3) should be provided or not for performing 3D-reconstruction based on evaluating a geometrical complexity value related to a geometrical form of the stenosis or the section of the vessel (3), including the steps of

   - providing at least one image (4) of the stenosis (1,2) or the section of the vessel (3),
   - gathering at least one geometrical feature value (11) of the stenosis (1,2) and/or the section of the vessel (3) from the at least one image,
   - gathering at least one intensity feature value (10) based on a grey level intensity of the stenosis (1,2) or the section of the vessel (3) from the at least one image (4),
   - automatically determining a complexity value (9) related to the geometrical complexity of the stenosis (1,2) or the section of the vessel (3) in dependency on the at least one geometrical feature value (11) and the at least one intensity feature value (10) of the stenosis (1,2) or the section of the vessel (3) and
   - using the complexity value (9) to determine whether the further image (4) of the stenosis (1,2) or the section of the vessel (3) should be provided or not.

2. Method according to claim 1, wherein the at least one image (4) is provided by angiography.

3. Method according to claim 1 or 2, wherein the at least one geometrical feature value relates to one of a 2D-contour, a centerline of a blood vessel or a curvature of the blood vessel.

4. Method according to one of the preceding claims, wherein the at least one intensity feature value results from an attenuation or excitation of radiation and relates to a sum, a distribution or an energy of grey values within one or more regions of the blood vessel (3) .

5. Method according to one of the preceding claims, wherein the complexity value (9) is determined by regression or classification of the at least one geometrical feature value and the at least one intensity feature value.

6. Method according to one of the preceding claims, wherein at least three feature values of one or more geometrical features and one or more intensity features are gathered and the complexity value (9) is determined by weighting the feature values.

7. Method according to one of the preceding claims, wherein a ratio of the at least one geometrical feature value (11) and the at least one intensity feature value (10) is used for determining the complexity value (9).

8. Method according to one of the preceding claims, wherein said automatically determining of the complexity value (9) is performed on the basis of machine learning.

9. Method according to one of the preceding claims, wherein the stenosis (1,2) is an eccentric stenosis.

10. Method according to one of the preceding claims, wherein the complexity value (9) is used to determine, whether a 3D-reconstruction of the stenosis (1,2) or the section of the vessel (3) is possible or not.

11. Device for determining whether a further image (4) of a stenosis (1,2) or a section of a vessel (3) should be provided or not for performing 3D-reconstruction based on evaluating a geometrical complexity value related to a geometrical form of the stenosis (1,2) or the section of the vessel (3), including

   - storage means for providing at least one image (4) of the stenosis (1,2) or the section of the vessel (3),

- analyzing means for gathering at least one geometrical feature value (11) of the stenosis (1,2) and/or or the section of the vessel (3) from the at least one image (4) and for gathering at least one intensity feature value (10) based on a grey level intensity of the stenosis (1,2) or the section of the vessel (3) from the at least one image,
- calculating means for automatically determining a complexity value (9) related to the geometrical complexity of the stenosis (1,2) or the section of the vessel (3) in dependency on the at least one geometrical feature value (11) and the at least one intensity feature value (10) of the stenosis (1,2) or the section of the vessel (3) and for using the complexity value (9) to determine whether the further image (4) of the stenosis (1,2) or the section of the vessel (3) should be provided or not.

**12.** Angiography system including a device according to claim 11.

**Patentansprüche**

**1.** Verfahren zum Bestimmen, ob ein weiteres Bild (4) einer Stenose (1, 2) oder einer Sektion eines Gefäßes (3) zum Durchführen einer 3D-Rekonstruktion vorgesehen werden soll oder nicht, basierend auf einem Auswerten eines geometrischen Komplexitätswertes, der sich auf eine geometrische Form der Stenose oder der Sektion des Gefäßes (3) bezieht, die folgenden Schritte umfassend:

- Vorsehen mindestens eines Bildes (4) der Stenose (1, 2) oder der Sektion des Gefäßes (3),
- Sammeln mindestens eines geometrischen Merkmalswertes (11) der Stenose (1, 2) und/oder der Sektion des Gefäßes (3) aus dem mindestens einen Bild,
- Sammeln mindestens eines Intensitätsmerkmalswertes (10) basierend auf einer Graustufenintensität der Stenose (1, 2) oder der Sektion des Gefäßes (3) aus dem mindestens einen Bild (4),
- automatisches Bestimmen eines Komplexitätswertes (9), der sich auf die geometrische Komplexität der Stenose (1, 2) oder der Sektion des Gefäßes (3) in Abhängigkeit von dem mindestens einen geometrischen Merkmalswert (11) und dem mindestens einen Intensitätsmerkmalswert (10) der Stenose (1, 2) oder der Sektion des Gefäßes (3) bezieht, und
- Verwenden des Komplexitätswertes (9), um zu bestimmen, ob das weitere Bild (4) der Stenose (1, 2) oder der Sektion des Gefäßes (3) vorgesehen werden soll oder nicht.

**2.** Verfahren nach Anspruch 1, wobei das mindestens eine Bild (4) durch eine Angiographie vorgesehen wird.

**3.** Verfahren nach Anspruch 1 oder 2, wobei sich der mindestens eine geometrische Merkmalswert auf eines von einer 2D-Kontur, einer Mittellinie eines Blutgefäßes oder einer Krümmung des Blutgefäßes bezieht.

**4.** Verfahren nach einem der vorhergehenden Ansprüche, wobei sich der mindestens eine Intensitätsmerkmalswert aus einer Schwächung oder Anregung einer Strahlung ergibt und sich auf eine Summe, eine Verteilung oder eine Energie von Grauwerten innerhalb eines oder mehrerer Bereiche des Blutgefäßes (3) bezieht.

**5.** Verfahren nach einem der vorhergehenden Ansprüche, wobei der Komplexitätswert (9) durch eine Regression oder Klassifizierung des mindestens einen geometrischen Merkmalswertes und des mindestens einen Intensitätsmerkmalswertes bestimmt wird.

**6.** Verfahren nach einem der vorhergehenden Ansprüche, wobei mindestens drei Merkmalswerte eines oder mehrerer geometrischer Merkmale und eines oder mehrerer Intensitätsmerkmale erfasst werden und der Komplexitätswert (9) durch ein Gewichten der Merkmalswerte bestimmt wird.

**7.** Verfahren nach einem der vorhergehenden Ansprüche, wobei ein Verhältnis des mindestens einen geometrischen Merkmalswertes (11) und des mindestens einen Intensitätsmerkmalswertes (10) zum Bestimmen des Komplexitätswertes (9) verwendet wird.

**8.** Verfahren nach einem der vorhergehenden Ansprüche, wobei das automatische Bestimmen des Komplexitätswertes (9) auf der Grundlage eines maschinellen Lernens durchgeführt wird.

**9.** Verfahren nach einem der vorhergehenden Ansprüche, wobei die Stenose (1, 2) eine exzentrische Stenose ist.

**10.** Verfahren nach einem der vorhergehenden Ansprüche, wobei der Komplexitätswert (9) verwendet wird, um zu bestimmen, ob eine 3D-Rekonstruktion der Stenose (1, 2) oder der Sektion des Gefäßes (3) möglich ist oder nicht.

11. Vorrichtung zum Bestimmen, ob ein weiteres Bild (4) einer Stenose (1, 2) oder einer Sektion eines Gefäßes (3) zum Durchführen einer 3D-Rekonstruktion vorgesehen werden soll oder nicht, basierend auf der Auswertung eines geometrischen Komplexitätswertes, der sich auf eine geometrische Form der Stenose (1, 2) oder der Sektion des Gefäßes (3) bezieht, enthaltend:

- Speichermittel zum Vorsehen mindestens eines Bildes (4) der Stenose (1, 2) oder der Sektion des Gefäßes (3),
- Analysemittel zum Sammeln mindestens eines geometrischen Merkmalswertes (11) der Stenose (1, 2) und/oder der Sektion des Gefäßes (3) aus dem mindestens einen Bild (4) und zum Sammeln mindestens eines Intensitätsmerkmalswertes (10) basierend auf einer Graustufenintensität der Stenose (1, 2) oder der Sektion des Gefäßes (3) aus dem mindestens einen Bild,
- Berechnungsmittel zum automatischen Bestimmen eines Komplexitätswertes (9), der sich auf die geometrische Komplexität der Stenose (1, 2) oder der Sektion des Gefäßes (3) in Abhängigkeit von dem mindestens einen geometrischen Merkmalswert (11) und dem mindestens einen Intensitätsmerkmalswert (10) der Stenose (1, 2) oder der Sektion des Gefäßes (3) bezieht, und zum Verwenden des Komplexitätswertes (9) zum Bestimmen, ob das weitere Bild (4) der Stenose (1, 2) oder der Sektion des Gefäßes (3) vorgesehen werden soll oder nicht.

12. Angiographiesystem mit einer Vorrichtung nach Anspruch 11.

**Revendications**

1. Procédé pour déterminer si une autre image (4) d'une sténose (1, 2) ou d'un tronçon d'un vaisseau (3) doit être prévue ou ne doit pas l'être pour effectuer une reconstruction en 3D reposant sur une évaluation d'une valeur de complexité géométrique se rapportant à une forme géométrique de la sténose ou du tronçon du vaisseau (3), comprenant les stades dans lesquels

- on se procure au moins une image (4) de la sténose (1, 2) ou du tronçon du vaisseau (3),
- on recueille au moins une valeur (11) de caractéristique géométrique de la sténose (1, 2) et/ou du tronçon du vaisseau (3) dans la au moins une image,
- on recueille au moins une valeur (10) de caractéristique d'intensité, reposant sur une intensité de niveau de gris de la sténose (1, 2) ou du tronçon du vaisseau (3) dans la au moins une image (4),
- on détermine automatiquement une valeur (9) de complexité, se rapportant à la complexité géométrique de la sténose (1, 2) ou du tronçon du vaisseau (3), en fonction de la au moins une valeur (11) de caractéristique géométrique et de la au moins une valeur (10) de caractéristique d'intensité de la sténose (1, 2) ou du tronçon du vaisseau (3) et
- on utilise la valeur (9) de complexité pour déterminer s'il faut prévoir ou non l'autre image (4) de la sténose (1, 2) ou du tronçon du vaisseau (3).

2. Procédé suivant la revendication 1, dans lequel on prévoit la au moins une image (4) par angiographie.

3. Procédé suivant la revendication 1 ou 2, dans lequel la au moins une valeur de caractéristique géométrique se rapporte à l'un d'un contour en 2D d'une ligne centrale d'un vaisseau sanguin ou d'une courbure du vaisseau sanguin.

4. Procédé suivant l'une des revendications précédentes, dans lequel la au moins une valeur de caractéristique d'intensité provient d'une atténuation ou d'une excitation de rayonnement et se rapporte à une somme, une répartition ou une énergie de valeurs de gris dans une ou plusieurs régions du vaisseau (3) sanguin.

5. Procédé suivant l'une des revendications précédentes, dans lequel on détermine la valeur (9) de complexité par régression ou classification de la au moins une valeur de caractéristique géométrique et de la au moins une valeur de caractéristique d'intensité.

6. Procédé suivant l'une des revendications précédentes, dans lequel on recueille au moins trois valeurs de caractéristique d'une ou de plusieurs caractéristiques géométriques et d'une ou de plusieurs caractéristiques d'intensité et on détermine la valeur (9) de complexité en pondérant les valeurs de caractéristique.

7. Procédé suivant l'une des revendications précédentes, dans lequel on utilise un rapport de la au moins une valeur (11) de caractéristique géométrique et de la au moins une valeur (10) de caractéristique d'intensité pour déterminer la valeur (9) de complexité.

8. Procédé suivant l'une des revendications précédentes, dans lequel on effectue automatiquement la détermination de la valeur (9) de complexité sur la base d'un apprentissage automatique.

9. Procédé suivant l'une des revendications précédentes, dans lequel la sténose (1, 2) est une sténose avec épaississement excentrique de la paroi.

10. Procédé suivant l'une des revendications précédentes, dans lequel on utilise la valeur (9) de complexité pour déterminer si une reconstruction en 3D de la sténose (1, 2) ou du tronçon du vaisseau (3) est possible ou ne l'est pas.

11. Dispositif pour déterminer si une autre image (4) d'une sténose (1, 2) ou d'un tronçon d'un vaisseau (3) doit être prévue ou ne doit pas l'être pour effectuer une reconstruction en 3D reposant sur une évaluation d'une valeur de complexité géométrique se rapportant à une forme géométrique de la sténose (1, 2) ou du tronçon du vaisseau (3), comprenant

- des moyens de mise en mémoire pour fournir au moins une image (4) de la sténose (1, 2) ou du tronçon du vaisseau (3),
- des moyens d'analyse pour recueillir au moins une valeur (11) de caractéristique géométrique de la sténose (1, 2) et/ou du tronçon du vaisseau (3) dans la au moins une image (4) et pour recueillir au moins une valeur (10) de caractéristique d'intensité reposant sur une intensité de niveau de gris de la sténose (1, 2) ou du tronçon du vaisseau (3) dans la au moins une image,
- des moyens de calcul pour déterminer automatiquement une valeur (9) de complexité se rapportant à la complexité géométrique de la sténose (1, 2) ou du tronçon du vaisseau (3), en fonction de la au moins une valeur (11) de caractéristique géométrique et de la au moins une valeur (10) de caractéristique d'intensité de la sténose (1, 2) ou du tronçon du vaisseau (3) et pour utiliser la valeur (9) de complexité, afin de déterminer si l'autre image (4) de la sténose (1, 2) ou du tronçon du vaisseau (3) doit être prévue ou ne doit pas l'être.

12. Système d'angiographie, comprenant un dispositif suivant la revendication 11.

# FIG 1

90°

1

3

2

# FIG 2

60°

1

3

2

# FIG 3

30°

1

3

2

# FIG 4

0°

1

3

2

# FIG 5

4

6    5    7

8

9

FIG 6

EP 3 270 355 B1

**REFERENCES CITED IN THE DESCRIPTION**

**Non-patent literature cited in the description**

- **ITU, LUCIAN et al.** A Machine Learning Approach for Computation of Fractional Flow Reserve from Coronary Computed Tomography. *Journal of Applied Physiology,* 14 April 2016

- Computerized detection of noncalcified plaques in coronary CT angiography: Evaluation of topological soft gradient prescreening method an luminal analysis. **JUN WEI et al.** Medical Physics. AIP, 01 August 2014, vol. 41